# EUROPEAN PATENT SPECIFICATION

(11) **EP 3 682 942 B1**
(45) Date of publication and mention of the grant of the patent: **25.01.2023**
(21) Application number: 20159887.7
(22) Date of filing: 22.12.2016
(51) Int. Cl.: A61N 1/32, H02J 7/00, A61B 18/12, A61B 18/00

(54) **MODULAR, VARIABLE ELECTRONIC POWER SYSTEM FOR GENERATING ELECTRICAL PULSES**
MODULARES, VARIABLES ELEKTRONISCHES LEISTUNGSSYSTEM ZUR ERZEUGUNG ELEKTRISCHER IMPULSE
SYSTÈME D'ALIMENTATION ÉLECTRONIQUE MODULAIRE ET VARIABLE POUR GÉNÉRER DES IMPULSIONS ÉLECTRIQUES

(30) Priority: 22.12.2015 ES 201531870
(43) Date of publication of application: 22.07.2020
(62) Divisional of application: 16877840.5
(73) Proprietor: Universidad De Zaragoza, 50009 Zaragoza (ES); Universitat Pompeu Fabra, 08080 Barcelona (ES)
(72) Inventor: SARNAGO ANDÍA, Héctor, 50009 Zaragoza (ES); LUCÍA GIL, Óscar, 50009 Zaragoza (ES); BURDÍO PINILLA, José Miguel, 50009 Zaragoza (ES); NAVAL PALLARÉS, Alejandro, 50009 Zaragoza (ES); IVORRA CANO, Antoni, 08080 Barcelona (ES); CASTELLVÍ FERNÁNDEZ, Quim, 08080 Barcelona (ES)
(74) Representative: Hoffmann Eitle

(56) References cited:
- WO-A1-2011/017802
- US-A1- 2007 242 743
- US-A1- 2011 065 161
- US-A1- 2015 155 716
- US-A1- 2015 263 526
- VARMA R ET AL: "Development of a solid state versatile pulsar for high voltage and high power applications", PULSED POWER CONFERENCE, 2009. PPC '09. IEEE, IEEE, PISCATAWAY, NJ, USA, 28 June 2009 (2009-06-28), pages 1312-1316, XP031615086, ISBN: 978-1-4244-4064-1
- REDONDO L ET AL: "Analysis of a modular generator for high-voltage, high-frequency pulsed applications, using low voltage semiconductors (<1kV) and series connected step-up (1:10) transformers", REVIEW OF SCIENTIFIC INSTRUMENTS, AIP, MELVILLE, NY, US, vol. 78, no. 3, 13 March 2007 (2007-03-13) , pages 34702-34702, XP012103847, ISSN: 0034-6748, DOI: 10.1063/1.2709743

## Description

### FIELD OF THE INVENTION

The present invention is comprised in the technical field of electroporation devices intended for use in medical treatments for improving the absorption of medicinal products or the destruction of tumor cells. More specifically, the invention relates to a modular, variable electronic power system for generating unipolar and bipolar electrical pulses. The sector of greatest interest of the invention is the biomedicine sector, although it is also applicable in other sectors, such as sterilization in the food industry.

### BACKGROUND OF THE INVENTION

Electroporation or electropermeabilization is a technique used in medicine which consists of applying a pulsed electric field to a living organism such that it experiences, at the cell membrane level, changes which may be temporary or permanent depending on the intensity of the applied field.

Said electrical pulses are produced by generators having different characteristics which vary depending on the electroporation technique to be used or on the problem to be solved. When the voltage going through a plasma membrane exceeds its dielectric rigidity, pores, which can close after a certain period of time, are formed. In the cases in which the opening of the pores is temporary and reversible, extracellular compounds may be introduced into the cell for therapeutic purposes. Alternatively, the pores can remain open irreversibly, giving rise to apoptotic cell death. In this context, the reversibility of the technique, as well as the size of the pores and the duration they remain open depend on the intensity of the applied electric field and on the time the cell is exposed to said field.

Irreversible electroporation (or "IRE") is a non-thermal ablation technique that is gaining a lot of interest today for the treatment of certain types of tumor with increased resistance. It consists of applying strong electric fields for the purpose of causing permeabilization of the cell membranes of the tissue to cause cell death. Some advantages of this technique with respect to conventional tumor ablation techniques are the possibility of treating regions close to large blood vessels since the thermal cooling will not affect them, or of preserving the connective tissue, blood vessels, and other ducts. To achieve irreversibility in the electroporation technique, the generator must reach a high voltage and current level, the threshold of which varies depending on the type of cells to be treated.

Unipolar pulse generators intended for medical applications today have insufficient maximum voltage levels for effective, widespread use in electroporation techniques. This is the case, for example, of the system disclosed in Review of Scientific Instruments 78, 034702 (2007), in the scientific paper entitled "Analysis of a modular generator for high-voltage, high-frequency pulsed applications, using low voltage semiconductors (1 kV) and series connected step-up (1:10) transformers" (L.M. Redondo *et al*.), which describes a modular generator that produces unipolar pulses which, despite being high voltage, are still below the voltage levels required for the applications described herein. Another similar case is the system disclosed in patent application WO 2011/017802 A1 (S. Jayaram et al.) which describes an electric generator having a plurality of modules connected in cascade and producing unipolar pulses having a variable output voltage, depending on the number of modules that are included in the system.

In this manner, even though the known modular generators allow solving some deficiencies of conventional techniques, they are limited in terms of the maximum voltages and currents that they can reach, and they also have severe constraints as regards the duration and configurability of the generated pulses, making the application thereof in the field of irreversible electroporation of tumor tissues difficult.

Likewise, existing generators for application thereof in irreversible electroporation which are capable of offering the required output voltages and currents are, however, rather non-versatile, where they allow obtaining a limited range of voltages and their use is likewise limited to certain specific types of cells or situations.

According to what has been described in the preceding paragraphs, there is a need in the present technical field for alternatives that allow solving the described problems for the purpose of attaining output voltage and current values suitable for widespread use in irreversible electroporation, as well as device versatility that allows adapting said device to a wide variety of medical situations or applications.

The present invention is intended to solve said problems by means of a novel modular system for generating high-voltage unipolar or bipolar electrical pulses as defined by the independent claim 1.

Technical publication: VARMA R ET AL, "Development of a solid state versatile pulsar for high voltage and high power applications", PULSED POWER CONFERENCE, 2009. PPC '09. IEEE, IEEE, PISCATAWAY, NJ, USA, (20090628), ISBN 978-1-4244-4064-1, pages 1312 - 1316, XP031615086 [A], relates to a solid state device for high voltage and high power, that provides independent variation of pulse amplitude, width and repletion rate.

### BRIEF DESCRIPTION OF THE INVENTION

An object of the present invention is therefore to provide a pulse generator technology based on a modular structure and unipolar or bipolar pulses, which allows greater versatility and higher output voltage than generators of the state of the art. To that end, the invention proposes a high-voltage generator based on a modular, versatile electronic power system which includes a control unit and allows adapting the intensity and other characteristics of the electrical pulses to each specific application, depending on the number of modules there are. Said generator is preferably applied in electroporation, where it can be adapted to different specific problems or organs given the versatility of the modular system and the capacity to achieve high voltage and current levels.

The mentioned object of the invention is preferably carried out by means of a system comprising:
- one or more electrical pulse generation modules, wherein said modules are connectable in series or in parallel. By means of the connection in series, the output voltage of said pulses is the sum of the individual output voltages of each module. By means of the connection in parallel, the total current is the sum of the currents of each module.
- a charging unit for the generation modules;
- a control unit for the generation modules and the charging unit.

Advantageously, the generation modules are coupled to the charging unit by means of isolation transformers, said charging unit being arranged as the primary of the transformers, and the generation modules as the secondaries of the transformers.

Likewise, each generation module preferably comprises an AC/DC rectifier connected to the output of its respective transformer, and a DC/AC inverter connected to said AC/DC rectifier in a bridge configuration for generating electrical output pulses or pulse trains; and the charging unit comprises a DC/DC step-up converter connected to an indirect DC/AC inverter, wherein said DC/AC inverter is connected to the input of the primary of the transformer.

Higher voltage and intensity in the pulses are obtained due to their bipolar nature and to the possibility of adding modules both in series and in parallel to the architecture of the device, which in turn raises a technical solution that adds versatility to the devices. Bipolar pulses are also achieved in the present invention as a result of the bridge configuration of the inverter inside each generation module.

More specifically, the generator of the invention allows obtaining pulses with a high voltage level (of the order of 10-15 kV peak to peak) and a high current level (400-600 A peak to peak), well exceeding generators that are available today in the clinical setting, and obtaining, in medical applications, more than twice the voltage and more than five times the current obtained with the technologies existing on the market. This means that with the generator of the invention, it is possible to achieve ablation volumes that are much greater than the volumes which can be achieved today, and by not using a low-frequency transformer, a more compact and lightweight solution compared to the solution offered by current generators is provided.

On the other hand, the modular design proposed by the system of the invention allows using the number of modules needed to achieve the voltage required in a given application. A greater versatility in the output voltage is thereby obtained by means of unipolar or bipolar pulses and pulse trains, having a width (from 1 µs) and number of pulses that are completely configurable. This configurability leads to the following technical advantages:
- Attenuating the effect of electrochemical reactions. These reactions are harmful both to the electrodes and to the organic tissues.
- Eliminating the formation of hydrogen and oxygen bubbles by hydrolysis.
- Less neurostimulation leading to unwanted muscle activation.
- Possibility of applying rapid bursts of short pulses, which significantly reduces total treatment time.

As an additional advantage, the system of the invention does not require using a transformer at the output. This constitutes a key difference as it obtains an output impedance that is much lower, and therefore less affected by the charge. This aspect is very important in electroporation as both the electrodes and the tissue to be connected are highly variable in terms of charge. The invention therefore allows assuring a square wave voltage form in the output at all times.

In a preferred embodiment of the invention, one or more pulse generation modules comprise an auxiliary AC/DC block, powered by the output of the isolation transformer, and likewise connected to the AC/DC rectifier and to the DC/AC inverter so as to generate a voltage for feeding same. In turn and in a similar manner, the charging unit preferably comprises an auxiliary DC/DC block, connected to the DC/DC step-up converter and to the indirect DC/AC inverter so as to generate a voltage for feeding same.

In another preferred embodiment of the invention, the frequency of the indirect DC/AC inverter of the charging unit is equal to or greater than 200 kHz, and the isolation voltage of the transformers is preferably equal to or greater than 15 kV.

In another preferred embodiment of the invention, the generator includes a control architecture based on a programmable logic device (FPGA) which allows the current and future implementation of advanced synchronization functions with ECG, protections, treatment automation, etc. A greater degree of versatility and adaptation of the pulses of the output voltage to the treatment to be performed is thereby achieved.

The control unit of the system of the invention likewise provides the capacity to program the number of active generation modules while applying the pulses. This allows quickly varying the magnitude of the applied pulses or pulse trains, thereby configuring the form thereof (for example, it is possible to apply pulses or pulse trains in the form of a step). This capacity is of interest, for example, in applications relating to electroporation-assisted gene transfection (or "gene electrotransfer"). It has been demonstrated in this field of application of electroporation that protocols consisting of a high-magnitude short pulse, followed by another low-magnitude long pulse, are more effective than two or more high-magnitude short pulses.

In another preferred embodiment of the invention, the control unit comprises at least one connection with the generation modules and at least one connection with the charging unit, said connections being isolated by means of optical fibers. Improved isolation which increases safety when using the system is thereby achieved.

In another preferred embodiment of the invention, the generator is powered by means of batteries, and not through direct connection to the power grid like in generators used today, thereby improving safety and isolation during use, and facilitating the standardization process and compliance with electromagnetic compatibility regulations.

In another preferred embodiment of the invention, the generator comprises a wireless communication subsystem for communication by means of a WiFi connection with a computer, through which various parameters such as polarity, amplitude, the number of pulses in each burst, the number of bursts, and their frequency of repetition, can be configured. This possibility of wireless control significantly increases safety and ease of application.

Another object of the present invention relates to the associated uses of the system, comprising applications for food sterilization, waste treatment, contamination control, metal or semiconductor treatment, molecular biology assays, and/or medical or cosmetic treatments. The uses of the system associated with molecular biology assays and medical and/or cosmetic treatments preferably comprise the applications of electroporation.

### DESCRIPTION OF THE DRAWINGS

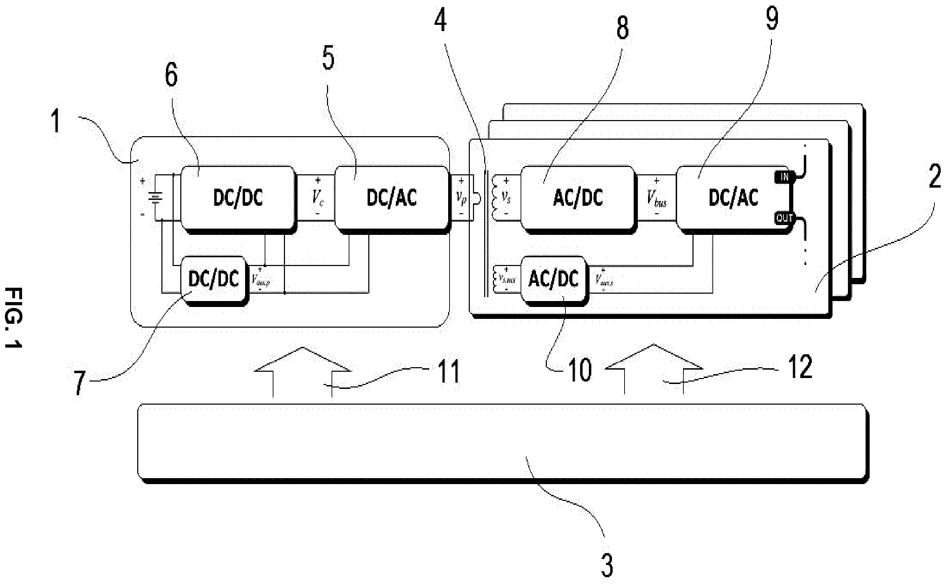

Figure 1 shows a block diagram of the modular, versatile electronic power system of the invention according to a preferred embodiment thereof.

### DETAILED DESCRIPTION OF THE INVENTION

A detailed description of the invention is provided below in reference to a preferred embodiment thereof based on Figure 1 herein. Said embodiment is provided to illustrate and not limit the claimed invention.

As described in the preceding sections, the high-voltage generator proposed by the present invention is based on a modular, versatile electronic power system which allows adapting the design to the output voltage and to the characteristics of the required pulses, depending on the specific application or treatment to be applied.

Said Figure 1 shows the general block diagram of the modular electronic power system of the invention, where said system essentially comprises a charging unit (1), one or more pulse generation modules (2) in a rectifier-inverter configuration, and a control unit (3). The pulse generation modules (2) are connected to the charging unit (1) by means of magnetic coupling through an isolation transformer (4).

The charging unit (1) is preferably formed by a high-frequency indirect DC/AC inverter (5), which is connected to an upstream DC/DC step-up converter (6). The main function of said charging unit (1) is to charge each of the generation modules (2) to the required voltage by means of the isolation transformer (4) by means of which said charging unit (1) and said generation modules (2) are coupled. It is important to highlight that the required isolation (greater than 15 kV) is achieved as a result of the coupling by means of the transformer (4), and a compact implementation of the system is achieved due to the high operating frequency (typically 200 kHz),.

In addition to the preceding elements, the charging unit (1) comprises an auxiliary DC/DC block (7) which is in charge of delivering a supply voltage V_{aux,p} for controlling the DC/AC inverter (5) and the DC/DC step-up converter (6).

In turn, the pulse generation modules (2), arranged in the secondary of the isolation transformer (4) of the system are in charge of generating the output voltage that will be applied during electroporation treatment. Each module (2) preferably consists of an AC/DC rectifier (8) and a DC/AC inverter (9), based on a bridge configuration, so as to have the capacity to generate bipolar output voltage pulses in each module (2).

Like the arrangement of the elements of the charging unit (1), each pulse generation module (2) can comprise an auxiliary AC/DC block (10), also powered from the secondary of the isolation transformer (4), which is in charge of generating the supply voltages V_{aux,s} of the AC/DC rectifier (8) and the DC/AC inverter (9).

The pulse generation modules (2) of the system of the invention can be interconnected in series, giving rise to an output voltage the value of which will be the sum of the voltages generated by each of the generation modules (2) separately. Likewise, the modules can be connected in parallel, so the delivered current will be the sum of the currents of each module. The invention thereby provides a variable power stage which allows being adapted to the needs of the treatment to be performed so as to generate the required voltage and current levels.

As described above, the system of the invention also comprises a control unit (3) which is in charge of controlling the electronic power system formed by the charging unit (1) and each of the pulse generation modules (2). The control signals of the generation modules (2) are preferably emitted using a programmable logic device (FPGA) integrated in said control unit (3). It is worth noting that the generation of the control signals by means of FPGA allows a greater degree of versatility and adaptation of the output voltage pulses to the treatment to be performed. This is not possible in current commercial systems which have severe constraints in terms of the types of voltage pulses they are capable of generating.

As mentioned in the preceding sections, the control unit (3) is preferably configured with programming means for programming the number of active generation modules (2) of the system while applying the pulses, which allows quickly varying the magnitude of the applied pulses or pulse trains, configuring the form thereof.

In a complementary manner, and due to the strict isolation requirements imposed by regulations governing the use and safety of electroporation devices, the control signals are preferably isolated by means of optical fibers (11, 12).

Finally, the system of the invention is preferably communicated through wireless means, for example by means of a WiFi network connected to a remote computer (not shown in Figure 1), through which the polarity, amplitude, the number of pulses in each burst, the number of bursts and their frequency of repetition, are configured.

The system of the invention provides satisfactory results both in the treatments of plant tissues and in the treatments of living animal tissues.

## Claims

1. A modular, variable electronic power system for generating unipolar or bipolar electrical pulses, comprising:
- electrical pulse generation modules (2), wherein said electrical pulse generation modules (2) are connectable in series or in parallel, such that output voltage and/or current of the generated unipolar or bipolar electrical pulses is respectively the sum of individual output voltages and/or currents of each electrical pulse generation module (2);
- a charging unit (1) for powering the electrical pulse generation modules (2); and
- a control unit (3) for controlling the electrical pulse generation modules (2) and the charging unit (1)
wherein each electrical pulse generation module (2) is coupled to the charging unit (1) by means of an isolation transformer (4) of the electrical pulse generation module (2), said charging unit (1) being connected to the primary side of the isolation transformers (4), and the electrical pulse generation modules (2) being connected to a secondary side of the isolation transformers (4);
wherein each electrical pulse generation module (2) comprises an AC/DC rectifier (8) connected to an output of the secondary side of the isolation transformer (4) of the electrical pulse generation module (2), **characterised in that** each electrical pulse generation module (2) comprises a DC/AC inverter (9) having a bridge configuration connected to an output of the AC/DC rectifier (8) of the electrical pulse generation module (2) for generating electrical output pulses or pulse trains.

2. The system according to claim 1, wherein the charging unit (1) comprises a DC/DC converter (6) connected to an DC/AC inverter (5), wherein said DC/AC inverter (5) is connected to the primary side of the isolation transformers (4).

3. The system according to claim 1 or 2, wherein one or more of said electrical pulse ieration modules (2) comprise an auxiliary AC/DC block (10), powered by the output of the secondary side of the isolation transformer (4) the one or more electrical pulse generation modules (2), and also connected to the AC/DC rectifier (8) and to the DC/AC inverter (9) of the one or more electrical pulse generation modules (2) so as to generate a voltage for feeding both.

4. The system according to claim 2, wherein the charging unit (1) comprises an auxiliary DC/DC block (7), connected to the DC/DC converter (6) and to the DC/AC inverter (5) so as to generate a voltage for feeding both.

5. The system according to claim 2 or 4, wherein the frequency of the DC/AC inverter (5) of the charging unit (1) is equal to or greater than 200 kHz.

6. The system according to any of the preceding claims, wherein the isolation voltage of the isolation transformers (4) is equal to or greater than 15 kV.

7. The system according to any of the preceding claims, wherein the control unit (3) is programmable to control the activation of the electrical pulse generation modules (2) while generating pulses, for varying the magnitude of applied pulses or pulse trains.

8. The system according to any of the preceding claims, wherein the control unit (3) comprises at least one connection with the electrical pulse generation modules (2) and at least one connection with the charging unit (1), said connections being isolated by means of optical fibers (11,12).

9. The system according to any of the preceding claims, wherein the control unit (3) is configured with programming means for programming the number of active electrical pulse generation modules (2) of the system during application of pulses.

10. The system according to any of the claims 2 to 9, comprising batteries for powering the charging unit (1), the electrical pulse generation modules (2), and the control unit (3).

11. The system according to the preceding claims, comprising at least one communication subsystem for communication with a computer, for configuration of polarity amplitude, number of pulses or pulse trains, and/or frequency of repetition thereof

12. The system according to the preceding claim, wherein the communication subsystem comprises a wireless connection with the computer through WiFi.

13. The system according to any of the preceding claims for use in applications for food sterilization, waste treatment, contamination control, metal or semiconductor treatment, molecular biology assays, and/or medical or cosmetic treatments.

14. The system according to any of the preceding claims for use in electroporation techniques.

15. The system according to any of the preceding claims, adapted to generate pulses having a voltage level in the order of 10 to 15 KV peak to peak, and a current level in the order of 400 to 600 Amps peak to peak.

## Patentansprüche

1. Modulares, variables elektronisches Leistungssystem zur Erzeugung unipolarer oder bipolarer elektrischer Impulse, umfassend:
- elektrische Impulserzeugungsmodule (2), wobei die genannten elektrischen Impulserzeugungsmodule (2) in Reihe oder parallelgeschaltet werden können, sodass die Ausgangsspannung und/oder der Ausgangsstrom der erzeugten unipolaren oder bipolaren elektrischen Impulse jeweils die Summe der einzelnen Ausgangsspannungen und/oder Ausgangsströme jedes elektrischen Impulserzeugungsmoduls (2) ist;
- eine Ladeeinheit (1) zur Versorgung der elektrischen Impulserzeugungsmodule (2); und
- eine Steuereinheit (3) zur Steuerung der elektrischen Impulserzeugungsmodule (2) und der Ladeeinheit (1);
wobei jedes elektrische Impulserzeugungsmodul (2) mittels eines Isoliertransformators (4) des elektrischen Impulserzeugungsmoduls (2) mit der Ladeeinheit (1) gekoppelt ist, wobei die genannte Ladeeinheit (1) an der Primärseite der Isoliertransformatoren (4) angeschlossen ist, und die elektrischen Impulserzeugungsmodule (2) an einer Sekundärseite der Isoliertransformatoren (4) angeschlossen sind;
wobei jedes elektrische Impulserzeugungsmodul (2) einen Wechselstrom/Gleichstrom-Gleichrichter (8) umfasst, welcher an einem Ausgang der Sekundärseite des Isoliertransformators (4) des elektrischen Impulserzeugungsmoduls (2) angeschlossen ist, **dadurch gekennzeichnet, dass** jedes elektrische Impulserzeugungsmodul (2) einen Gleichstrom/Wechselstrom-Wechselrichter (9) umfasst, welcher eine Brückenausbildung aufweist, welche an einem Ausgang des Wechselstrom/Gleichstrom-Gleichrichters (8) des elektrischen Impulserzeugungsmoduls (2) zur Erzeugung elektrischer Ausgangsimpulse oder -impulsfolgen angeschlossen ist.

2. System nach Anspruch 1, wobei die Ladeeinheit (1) einen Gleichstrom/Gleichstrom-Wandler (6) umfasst, welcher an einem Gleichstrom/Wechselstrom-Wechselrichter (5) angeschlossen ist, wobei der genannte Gleichstrom/Wechselstrom-Wechselrichter (5) an der Primärseite der Isoliertransformatoren (4) angeschlossen ist.

3. System nach Anspruch 1 oder 2, wobei eines oder mehrere der genannten elektrischen Impulserzeugungsmodule (2) einen Wechselstrom/Gleichstrom-Hilfsblock (10) umfassen, welcher vom Ausgang der Sekundärseite des Isoliertransformators (4) des einen oder der mehreren elektrischen Impulserzeugungsmodule (2) versorgt wird und auch am Wechselstrom/Gleichstrom-Gleichrichter (8) und am Gleichstrom/Wechselstrom-Wechselrichter (9) des einen oder der mehreren elektrischen Impulserzeugungsmodule (2) angeschlossen ist, um eine Spannung zur erzeugen, um beide zu speisen.

4. System nach Anspruch 2, wobei die Ladeeinheit (1) einen Gleichstrom/Gleichstrom-Hilfsblock (7) umfasst, welcher am Gleichstrom/Gleichstrom-Wandler (6) und am Gleichstrom/Wechselstrom-Wechselrichter (5) angeschlossen ist, um eine Spannung zu erzeugen, um beide zu speisen.

5. System nach Anspruch 2 oder 4, wobei die Frequenz des Gleichstrom/Wechselstrom-Wechselrichters (5) der Ladeeinheit (1) gleich oder größer als 200 kHz ist.

6. System nach einem der vorhergehenden Ansprüche, wobei die Isolationsspannung der Isoliertransformatoren (4) gleich oder größer als 15 kV ist.

7. System nach einem der vorhergehenden Ansprüche, wobei die Steuereinheit (3) programmierbar ist, um die Aktivierung der elektrischen Impulserzeugungsmodule (2) zu steuern, während sie Impulse erzeugen, um die Größe der angelegten Impulse oder -impulsfolgen zu ändern.

8. System nach einem der vorhergehenden Ansprüche, wobei die Steuereinheit (3) mindestens einen Anschluss an den elektrischen Impulserzeugungsmodulen (2) und mindestens einen Anschluss an der Ladeeinheit (1) umfasst, wobei die genannten Anschlüsse mittels optischer Fasern (11, 12) isoliert sind.

9. System nach einem der vorhergehenden Ansprüche, wobei die Steuereinheit (3) mit Programmiermitteln ausgebildet ist, um die Anzahl von aktiven elektrischen Impulserzeugungsmodulen (2) des Systems während des Anlegens der Impulse zu programmieren.

10. System nach einem der Ansprüche 2 bis 9, umfassend Batterien zur Versorgung der Ladeeinheit (1), der elektrischen Impulserzeugungsmodule (2) und der Steuereinheit (3).

11. System nach den vorhergehenden Ansprüchen, mindestens umfassend ein Kommunikationsteilsystem zum Kommunizieren mit einem Computer, zur Ausbildung der Polarität, Amplitude, Anzahl von Impulsen oder Impulsfolgen und/oder Wiederholungsfrequenz derselben.

12. System nach dem vorhergehenden Anspruch, wobei das Kommunikationsteilsystem einen drahtlosen Anschluss am Computer über WLAN umfasst.

13. System nach einem der vorhergehenden Ansprüche für dessen Verwendung in Anwendungen zur Sterilisation von Lebensmitteln, Abfallbehandlung, Kontaminationskontrolle, Metall- oder Halbleiterbehandlung, zu Molekularbiologieversuchen und/oder zu medizinischen oder kosmetischen Behandlungen.

14. System nach einem der vorhergehenden Ansprüche für dessen Verwendung in Elektroporationstechniken.

15. System nach einem der vorhergehenden Ansprüche, welches dazu angepasst ist, Impulse zu erzeugen, welche einen Spannungspegel von ungefähr 10 bis 15 KV von Spitze zu Spitze, und einen Strompegel von ungefähr 400 bis 600 Amps von Spitze zu Spitze aufweisen.

## Revendications

1. Système d'alimentation électronique modulaire et variable pour générer des impulsions électriques unipolaires ou bipolaires, comprenant :
- des modules de génération d'impulsions électriques (2), dans lequel lesdits modules de génération d'impulsions électriques (2) peuvent être connectés en série ou en parallèle, de telle sorte que la tension et/ou le courant de sortie des impulsions électriques unipolaires ou bipolaires générées est respectivement la somme de tensions et/ou de courants de sortie individuelles de chaque module de génération d'impulsions électriques (2) ;
- une unité de charge (1) pour l'alimentation des modules de génération d'impulsions électriques (2) ; et
- une unité de contrôle (3) pour le contrôle des modules de génération d'impulsions électriques (2) et de l'unité de charge (1) ;
dans lequel chaque module de génération d'impulsions électriques (2) est couplé à l'unité de charge (1) au moyen d'un transformateur d'isolement (4) du module de génération d'impulsions électriques (2), ladite unité de charge (1) étant connectée au côté primaire des transformateurs d'isolement (4), et les modules de génération d'impulsions électriques (2) étant connectés à un côté secondaire des transformateurs d'isolement (4) ;
dans lequel chaque module de génération d'impulsions électriques (2) comprend un redresseur CA/CC (8) connecté à une sortie du côté secondaire du transformateur d'isolement (4) du module de génération d'impulsions électriques (2), **caractérisé en ce que** chaque module de génération d'impulsions électriques (2) comprend un onduleur CC/CA (9) ayant une configuration en pont connectée à une sortie du redresseur CA/CC (8) du module de génération d'impulsions électriques (2) pour générer des impulsions de sortie électriques ou des trains d'impulsions.

2. Système selon la revendication 1, dans lequel l'unité de charge (1) comprend un convertisseur CC/CC (6) connecté à un onduleur CC/CA (5), dans lequel ledit onduleur CC/CA (5) est connecté au côté primaire des transformateurs d'isolement (4).

3. Système selon la revendication 1 ou 2, dans lequel un ou plusieurs desdits modules de génération d'impulsions électriques (2) comprennent un bloc CA/CC auxiliaire (10), alimenté par la sortie du côté secondaire du transformateur d'isolement (4) de l'un ou plusieurs modules de génération d'impulsions électriques (2), et également connecté au redresseur CA/CC (8) et à l'onduleur CC/CA (9) de l'un ou plusieurs modules de génération d'impulsions électriques (2) de manière à générer une tension pour l'approvisionnement des deux.

4. Système selon la revendication 2, dans lequel l'unité de charge (1) comprend un bloc CC/CC auxiliaire (7), connecté au convertisseur CC/CC (6) et à l'onduleur CC/CA (5) de manière à générer une tension pour l'approvisionnement des deux.

5. Système selon la revendication 2 ou 4, dans lequel la fréquence de l'onduleur CC/CA (5) de l'unité de charge (1) est égale ou supérieure à 200 kHz.

6. Système selon l'une quelconque des revendications précédentes, dans lequel la tension d'isolement des transformateurs d'isolement (4) est égale ou supérieure à 15 kV.

7. Système selon l'une quelconque des revendications précédentes, dans lequel l'unité de contrôle (3) est programmable pour contrôler l'activation des modules de génération d'impulsions électriques (2) tout en générant des impulsions, pour la variation de la magnitude des impulsions ou des trains d'impulsions appliquées.

8. Système selon l'une quelconque des revendications précédentes, dans lequel l'unité de contrôle (3) comprend au moins une connexion avec les modules de génération d'impulsions électriques (2) et au moins une connexion avec l'unité de charge (1), lesdites connexions étant isolées au moyen de fibres optiques (11, 12).

9. Système selon l'une quelconque des revendications précédentes, dans lequel l'unité de contrôle (3) est configurée avec des moyens de programmation pour la programmation du nombre de modules de génération d'impulsions électriques actifs (2) du système pendant l'application d'impulsions.

10. Système selon l'une quelconque des revendications 2 à 9, comprenant des batteries pour l'alimentation de l'unité de charge (1), des modules de génération d'impulsions électriques (2), et de l'unité de contrôle (3).

11. Système selon les revendications précédentes, comprenant au moins un sous-système de communication pour la communication avec un ordinateur, pour la configuration de l'amplitude de polarité, du nombre d'impulsions ou de trains d'impulsions et/ou de la fréquence de répétition de ceux-ci.

12. Système selon la revendication précédente, dans lequel le sous-système de communication comprend une connexion sans fil avec l'ordinateur par WiFi.

13. Système selon l'une quelconque des revendications précédentes pour son utilisation dans des applications pour stérilisation d'aliments, traitement de déchets, contrôle de pollution, traitement de métal ou de semiconducteur, essais de biologie moléculaire, et/ou traitements médicaux ou cosmétiques.

14. Système selon l'une quelconque des revendications précédentes pour son utilisation dans des techniques d'électroporation.

15. Système selon l'une quelconque des revendications précédentes, adapté pour générer des impulsions ayant un niveau de tension de l'ordre de 10 à 15 KV de crête à crête, et un niveau de courant de l'ordre de 400 à 600 A de crête à crête.
